# EUROPÄISCHE PATENTSCHRIFT

(11) **EP 3 500 216 B1**
(45) Veröffentlichungstag und Bekanntmachung des Hinweises auf die Patenterteilung: **28.07.2021**
(21) Anmeldenummer: 18772731.8
(22) Anmeldetag: 05.09.2018
(51) Int. Cl.: A61F 2/44, A61F 2/30, A61F 2/46

(54) **ZWISCHENWIRBEL-IMPLANTAT**
INTERVERTEBRAL IMPLANT
IMPLANT INTERVERTÉBRAL

(30) Priorität: 13.09.2017 DE 102017008592
(43) Veröffentlichungstag der Anmeldung: 26.06.2019
(73) Patentinhaber: Joimax GmbH, 76227 Karlsruhe (DE)
(72) Erfinder: BARTHOLD, Clemens, 76133 Karlsruhe (DE); RIES, Wolfgang, 76351 Linkenheim (DE); DÜRR, Alexander, 71706 Markgröningen (DE)
(74) Vertreter: Lichti - Patentanwälte Partnerschaft mbB
(86) Internationale Anmeldenummer: PCT/EP2018/000426
(87) Internationale Veröffentlichungsnummer: WO 2019/052681

(56) Entgegenhaltungen:
- DE-C1- 10 113 689
- DE-U1-202014 003 441
- US-A1- 2004 199 251
- US-A1- 2010 152 853
- US-A1- 2014 031 943

## Beschreibung

Die Erfindung betrifft ein Zwischenwirbel-Implantat nach dem Oberbegriff des Anspruchs 1.

Zwischenwirbel-Implantate werden auch als Zwischenwirbel-Körbchen oder im Englischen als Interbody Cages bezeichnet. Bei einer Reihe von Wirbelsäulenschäden, insbesondere Bandscheibenschäden, wie Wirbelgleiten und Instabilität nach einem Bandscheibenvorfall; durch Stenose und Degeneration wird eine Versteifung der Wirbelsäule zwischen den beiden von dem Schaden betroffenen Wirbelkörpern durchgeführt. Hierzu werden Implantate nach Entfernen einer beschädigten und/oder (nerven-)schädigenden Bandscheibe aus dem Zwischenwirbelraum oder Bandscheibenfach zwischen zwei unmittelbar übereinander befindlichen Wirbeln der Wirbelsäule eingesetzt, um diese auf dem vorgegebenen Abstand zu halten. Es erfolgt eine Versteifung der Wirbelsäule zumindest im Bereich dieser beiden Wirbel. Dies wird als z.B. (lumbale) Wirbelfusion (Lumbar Interbody Fusion - LIF) bezeichnet. Gegebenenfalls werden die Wirbel noch - in minimalinvasiver Spinalchirurgie - mit einer Stab-Schrauben-Einheit, Facettengelenksschrauben oder translaminären Schrauben versehen und gegen das zwischen ihnen liegende Implantat verspannt. Insofern ist es auch wünschenswert, dass die Knochen mit dem Implantat verwachsen. Ein solches wird daher in der Regel mit einer rauen Oberfläche versehen.

Ein gattungsgemäßes Zwischenwirbel-Implantat ist beispielsweise aus der DE 20 2014 003 441 U bekannt.

Die US 2014/0031943 A1 zeigt mit einem länglichen Körper mit einer vorderen Endnase, die die Seitenwände verbindet, um das Einführen des Abstandhalters in einen Bandscheibenraum zwischen Wirbeln in einer Einsetzrichtung zu erleichtern, von der aus der Abstandhalter dann gedreht wird, um die oberen und unteren Lagerflächen in Kontakt mit Endplatten benachbarter Wirbel zu positionieren. Die vordere Endnase bildet eine stumpfe konvexe Nase zwischen einer oberen und der unteren Lagerfläche, um den Lagerflächenbereich zu maximieren, der für den Kontakt mit den benachbarten Endplatten verfügbar ist.

Die US 2010/0152853 A1 zeigt ein Implantat mit einer biplanaren Angulation, das in einen Scheibenbereich unter Verwendung eines posterolateralen Ansatzes eingeführt werden kann.

Die US 2004/0199251 A1 zeigt einen Zwischenwirbelkörperabstandshalter mit konkaven Seitenflächen, die sich unter einem Winkel in Bezug auf die Längsachse erstrecken. Der Abstandhalter hat obere und untere konvexe Oberflächen, die durch einen Bogen definiert sind, der sich von einem Mittelpunkt ausgehend insbesondere vom Abstandhaltermittelpunkt erstreckt.

Die DE 101 13 689 C1 zeigt ein Zwischenwirbelimplantat mit Stützflächen an Außenseiten von Armen für benachbarte Wirbelkörper. An einem Ende sind die Arme durch eine Brücke verbunden, während am anderen Ende ein Distanzelement zum Aufspreizen der Arme angeordnet ist, das ein Exzenter ist, der um eine parallel zu den Armen verlaufende Drehachse verdrehbar derart angeordnet ist, dass er je nach seiner Winkelstellung die Arme mehr oder weniger aufspreizt.

Der Erfindung liegt die Aufgabe zugrunde, ein gattungsgemäßes Zwischenwirbel-Implantat dahingehend weiterzubilden, dass eine verbesserte Anpassung desselben an die benachbarten Wirbelkörper gegeben ist.

Erfindungsgemäß wird die genannte Aufgabe mit einem gattungsgemäßen Zwischenwirbel-Implantat dadurch gelöst, das kennzeichnende Merkmale des Anspruchs 1 aufweist.

Das Implantat ist derart - hinsichtlich seiner Höhe - asymmetrisch gegenüber seiner Vertikalmittelebene ausgebildet. Hierdurch wird die Anpassung an die natürlichen Gegebenheiten der oberen bzw. unteren Stirnseite von Wirbeln, zwischen denen das Implantat angeordnet wird, weiter unterstützt. Ein Implantat kann nicht zentral zwischen Wirbel eingebracht werden und ist daher zwischen diesen nicht in Richtung der Sagittalebene bzw. der Sagittalachse von posterior oder dorsal nach anterior oder ventral ausgerichtet. Das Implantat wird vielmehr in der Regel durch das Foramen intervertebrale eingebracht und liegt damit zur Sagittal-Achse/Ebene unter einem endlichen Winkel in der Größenordnung von etwa 45°. Durch die unterschiedliche Höhe an beiden Seiten des Implantats (mit einem stetigen Übergang zwischen diesen) erfolgt damit eine bessere Anpassung an die Lagesituation des Implantats zwischen den Wirbeln. Dabei ist insbesondere vorgesehen, dass die Höhe des Implantats an der einen Seite 2 mm geringer ist als an der anderen Seite. Implantate werden üblicherweise derart zwischen Wirbeln eingebracht, dass sie sich von - bei Sicht auf den Patienten - dorsal-links nach ventral-rechts erstrecken. In diesem Falle weist das Implantat in der Sicht von der seitlichen proximalen Stirnseite her (genauer Ausgangsfläche) in distaler Richtung eine linke Seite mit einer geringeren Höhe als die rechte Seite auf.

Das Implantat ist zum Einführen mittels minimal-invasiven Operationen durch das Foramen intervertebrale als Einzelimplantat in den Zwischenwirbelraum zwischen zwei Wirbeln ausgebildet. Es erstreckt sich daher im Zwischenwirbelraum mit endlichen Winkeln (Winkel ungleich 0°) sowohl zur Sagittal- als auch zur Frontalebene. Demgemäß ist Gegenstand zur Lösung der Aufgabe auch ein Verfahren zum Einbringen eines Zwischenwirbel-Implantats mit einem Stützabschnitt und mit einem sich an diesen über einen Übergangsbereich anschließenden proximalen Angriffsbereich, wobei Oberseite und Unterseite des Zwischenwirbel-Implantats symmetrisch zu einer Horizontalmittelebene ausgebildet sind, dadurch gekennzeichnet, dass es minimal-invasiv durch ein Foramen intervertebrale zwischen zwei Wirbeln derart als Einzelimplantat in den Wirbelzwischenraum eingebracht wird, dass es mit endlichen Winkeln sowohl einer Sagittal- als auch einer Frontalebene zwischen den Wirbeln zu liegen kommt.

Durch die symmetrische Ausgestaltung wird dabei die gewünschte verbesserte Anpassung des Implantats an die benachbarten Wirbelkörper erreicht.

Eine Weiterbildung sieht vor, dass zwischen Stützabschnitt und Angriffsabschnitt ein Übergangsbereich ausgebildet ist.

Zur weiteren Unterstützung ist dabei in bevorzugter Ausgestaltung vorgesehen, dass die Höhe des Stützabschnitts zwischen Übergangsbereich zum Angriffsabschnitt und einer dem Angriffsabschnitt abgewandten distalen Stirnseite größer ist als die Höhe beim Übergangsbereich und an der distalen Stirnseite, wobei insbesondere die größte Höhe in der Mitte zwischen dem Übergangsbereich zum Angriffsbereich und der dieser abgewandten distalen Stirnseite ausgebildet ist.

Eine Weiterbildung sieht vor, dass der Übergangsbereich zwischen Stützabschnitt und Angriffsabschnitt konkav ausgebildet ist. Durch die erfindungsgemäßen Ausbildungen des Implantats können die Wirbel zwischen denen es angeordnet ist optimal auf der Ober- und Unterseite des Implantats in allen Richtungen abrollen und gewähren daher trotz des eingesetzten Implantats eine Beweglichkeit der Wirbelsäule in diesem Bereich, wie sie bei bekannten Implantaten nicht gegeben ist.

Der Winkel zwischen Tangenten an die proximale Kante des Implantats zur distalen Stirnseite hin und an dem mittleren Bereich größter Höhe des Implantats liegt in Anpassung an den entsprechenden Lordosewinkel eines Patienten vorzugsweise zwischen 10° und 20°, insbesondere bei 12° bis 18°, also zur Längsmittelebene des Implantats liegt jede dieser Tangenten unter einem Winkel von 5° bis 10° bzw. in bevorzugterer Ausgestaltung zwischen 6° und 9°. Hierdurch wird die natürliche Lordose der Lendenwirbelsäule unterstützt bzw. wieder hergestellt. In bevorzugter Weise wird die (Maximal-)Höhe des Implantats an die körperliche Struktur der Wirbelsäule des Patienten bzw. dem entsprechenden Zwischenwirbelbereich, in den das Implantat angebracht werden soll, angepasst und kann dementsprechend eine angepasste Höhe im Bereich von 8 mm bis 18 mm, vorzugsweise 10 mm bis 16 mm aufweisen. Entsprechendes gilt für die Länge des Implantats, die 25 mm bis 35 mm, insbesondere zwischen 27 mm und 32 mm betragen sollte.

In weiterer bevorzugter Ausgestaltung ist ein äußerer Rahmen aus massiven Tragteilen und einem inneren Kern in Form eines Gitterkörpers vorgesehen, wobei insbesondere der Gitterkörper nur an parallel in einer Querrichtung verlaufenden Flächen mit dem Rahmen verbunden ist, aber in zu diesen Flächen unter einem endlichen Winkel verlaufenden Flächen und Kanten nicht mit dem Rahmen in Verbindung steht.

Die Herstellung eines solchen Implantats geschieht vorzugsweise durch Sintern, insbesondere mittels (selektiven) Elektronenstrahlschmelzen ((Selective) Electron Beam Melting, (S)EBM) oder mittels Lasersintern.(LST). Demgemäß ist das Implantat durch Sintern wie mittels Elektronenstrahlschmelzen oder Lasersintern, hergestellt.

Die netz- oder gitterförmigen Bereiche reichen vorzugsweise von einer Ober- oder Seitenfläche bis zur parallelen gegenüberliegenden Unter- bzw. Seitenfläche. Hierdurch wird erreicht, dass Knochengewebe nicht nur äußerlich oberflächlich an den Implantaten anwächst, sondern dieses in die Hohlräume der gitter- oder netzartigen Struktur einwächst und das Implantat so vollständig durchdringen kann bzw. das Knochenwachstum gefördert wird. Hierdurch wird eine feste Verbindung zwischen Implantat und benachbartem Wirbelkörper erreicht.

Durch die Ausbildung des Implantats mit zwei Strukturelementen, nämlich einem - äußeren - Rahmen aus kompaktem Material und in kompakter Struktur und einem inneren Gitterkörper mit der genannten netz- oder gitterartigen Struktur wird bei Beibehaltung der vorgenannten Vorteile des vollständigen Durchwachsens von Knochenmaterial durch das Implantat bzw. dessen gitterartige Struktur dem Implantat eine hinreichende Festigkeit und Steifigkeit verliehen.

Zudem ist der Gitterkörper nur an parallel in einer Richtung, nämlich Querrichtung verlaufenden Flächen mit dem Rahmen verbunden, aber in zu diesen Flächen unter einem endlichen Winkel verlaufenden Flächen und Kanten nicht mit dem Rahmen in Verbindung steht. Hierdurch wird in gewisser Weise eine Entkopplung des äußeren Rahmens und des Gitterkörpers des Implantats erreicht, indem in der Längs- oder Haupterstreckungsrichtung des Implantats (das Implantat ist in Einführrichtung länger als in Querrichtung und in seiner Höhe) beide entkoppelt sind. Wenn der Rahmen beispielsweise im Bereich von ihm bildenden Rippen unter auf ihn ausgeübten Druck nachgibt, so wird dieser nicht auf den Gitterkörper übertragen. Letzterer bleibt damit unbeeinträchtigt und auch in ihn eingewachsene Knochenstruktur wird nicht beeinträchtigt oder beschädigt.

Dabei ist in bevorzugter Weiterbildung vorgesehen, dass obere und untere Flächen des Gitterkörpers gleiche Abmessungen aufweisen, wie von Rahmenkomponenten umgebende Freiräume, die die genannten Flächen des Gitterkörpers umgeben - gegebenenfalls unter Berücksichtigung von Toleranzen von bis zu 0,3 mm.

In weiterer Ausbildung des erfindungsgemäßen Implantats ist vorgesehen, dass der Rahmen einen Hohlraum umgibt, in dem der Gitterkörper angeordnet ist. Eine Weiterbildung sieht vor, dass der Rahmen in seiner Längsrichtung verlaufende Längsrippen aufweist. Eine äußerst bevorzugte Ausgestaltung sieht vor, dass benachbarte Längsrippen mittig durch Querrippen verbunden sind. Hierdurch wird auch bei längeren Implantaten die Stabilität des Rahmens und damit des Implantats selbst erhöht. Die Netz- oder Gitterstruktur des Gitterkörpers des Implantats kann in verschiedener Weise ausgestaltet sein. In äußerst bevorzugter Ausgestaltung ist vorgesehen, dass der Gitterbereich oder Gitterkörper eine Diamantstruktur aufweist.

Auch kann vorgesehen sein, dass es einen durchgehenden Durchlass aufweist. Der Durchlass erstreckt sich dabei in Längsrichtung durch den Rahmen und insbesondere auch durch den Gitterkörper selbst. Hierdurch wird erreicht, dass das Implantat - mittels eines Einsetzinstruments über einen bis in den Zwischenwirbelraum oder das Bandscheibenfach liegenden Führungsdraht - durch eine rohrförmige Schleuse oder ein Endoskoprohr - eingeführt werden kann.

Eine bevorzugte Ausgestaltung des erfindungsgemäßen Implantats zeichnet sich dadurch aus, dass die gitterförmigen Bereiche oder der Gitterkörper Gitter-Öffnungsdurchmesser jeder Öffnung von 0,5 mm bis 3,5 mm aufweisen, dabei vorzugsweise an der Außenseite des Implantats Öffnungsdurchmesser jeder Öffnung in der Größenordnung von 0,5 mm bis 0,7 mm aufweisen.

Ein Implantat kann - wie gesagt - insbesondere durch (selektives) Elektronenstrahlschmelzen ((Selective) Electron Beam Melting (S)EBM) oder Lasersinterungstechnik (LST) aus Titanlegierung, insbesondere Ti6Al4V gemäß ISO 5832-3 hergestellt werden. Das Bauteil - Implantat - wird durch Aufschmelzen von Metallpulvermittels eines Elektronenstrahls oder Laserstrahls im Hochvakuum hergestellt. Hierdurch sind Hinterschneidungen ohne verlorene Formen oder Kerne erzeugbar. Durch einen Elektronenstrahl oder Laserstrahl als Energiequelle wird dabei das Metallpulver gezielt aufgeschmolzen, wodurch kompakte Bauteile nahezu beliebiger Geometrie direkt aus Konstruktionsdaten hergestellt werden können. Es wird abwechselnd eine Pulverlage mittels eines Rakels auf die vorherige aufgebracht und mittels des Elektronenstrahls belichtet. Auf diese Weise wird das gewünschte Bauteil schichtweise generiert.

Eine weitere Ausbildung des erfindungsgemäßen Verfahrens sieht vor, dass das Implantat derart in den Wirbelzwischenraum eingebracht wird, dass es dort unter einem Winkel zwischen 40° und 50°, vorzugsweise 45°, zur Sagittalebene zu liegen kommt. In bevorzugter Ausbildung ist vorgesehen, dass das Verfahren ein erfindungsgemäßes Implantat verwendet, das ein oder mehrere der oben beschriebenen Ausgestaltungen des Implantats aufweist.

Weitere Vorteile und Merkmale der Erfindung ergeben sich aus den Ansprüchen und aus der nachfolgenden Beschreibung, in der ein Ausführungsbeispiel des erfindungsgemäßen Implantats unter Bezugnahme auf die Zeichnung im Einzelnen erläutert ist. Dabei zeigt:
- Fig. 1: Die Anordnung eines erfindungsgemäßen Implantats zwischen zwei Wirbeln in Sicht der Horizontalachse;
- Fig. 2: die Anordnung eines erfindungsgemäßen Implantats zwischen zwei Wirbeln in dorsaler Sicht entlang der Sagittalachse;
- Fig. 3: eine Draufsicht von oben auf ein auf einen unteren Wirbel aufliegendes Implantat;

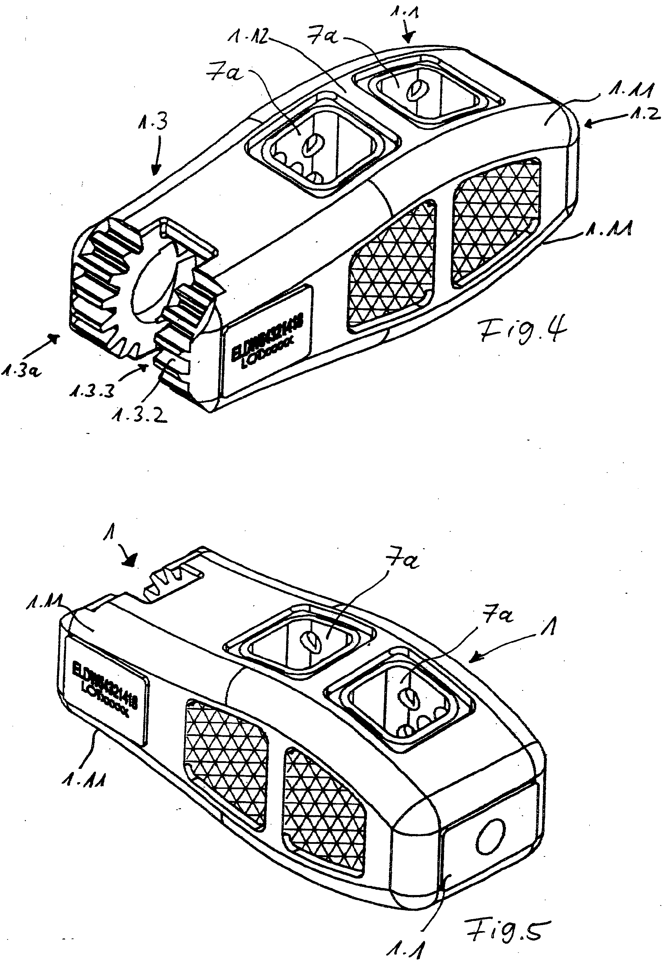

- Fig. 4: eine perspektivische Sicht der Außenkontur eines Implantats vom proximalen Ende her;
- Fig. 5: eine perspektivische Sicht der Außenkontur des Implantats vom distalen Ende her;
- Fig. 6: eine Draufsicht auf das Implantat von oben;
- Fig. 7: eine Seitenansicht des Implantats;
- Fig. 8: eine Ansicht der distalen Stirnseite des Implantats;
- Fig. 9: eine stirnseitige Sicht auf den Angriffsabschnitt des Implantats; und
- Fig. 10: eine perspektivische Darstellung des Gitterkranzes des Implantats.

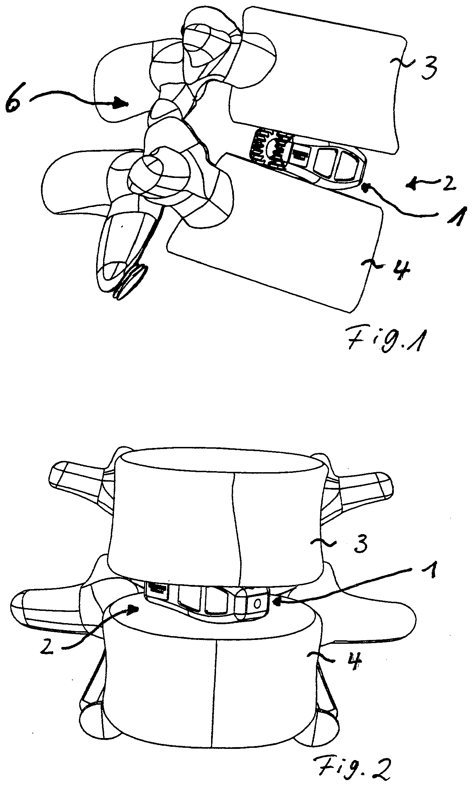

Wenn aufgrund von Schäden einer Bandscheibe der Wirbelsäule eines Patienten die Bandscheibe aus dem Zwischenwirbelraum 2 zwischen zwei übereinanderliegenden Wirbeln 3, 4 zu entfernen ist, wird stattdessen ein Zwischenwirbel-Implantat 1 eingesetzt, um die Wirbel 3, 4 auf geeignetem Abstand und in geneigter Ausrichtung zueinander zu halten, wie dies in den Fig. 1 und 2 dargestellt ist. Das Implantat 1 ist so ausgebildet, dass es mit den Wirbeln 3, 4 verwachsen kann.

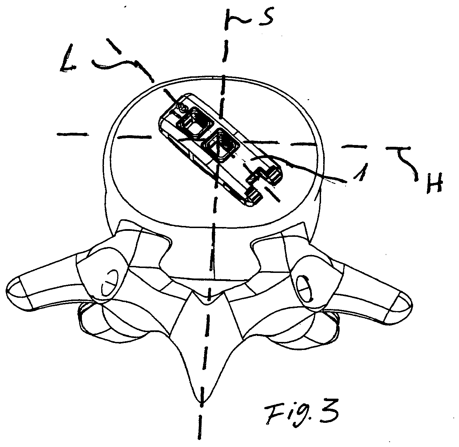

Das erfindungsgemäße Implantat 1 wird minimal-invasiv über einen - erweiterten - Zugang durch das - hier: rechte - Foramen intervertebrale 6 zwischen die beiden Wirbel 3, 4 eingebracht und liegt demgemäß, wie dies aus der Fig. 3 ersichtlich ist, als Einzelimplantat mit seiner Längsmittelachse L, unter einem Winkel von ca. 45° zur Sagittalachse S des Patientenkörpers bzw. der Wirbelsäule desselben, also mit seiner Längsmittelachse L etwa entlang der Winkelhalbierenden zwischen Horizontal- und Sagittalachse H, S des Körpers des Patienten. Während das Implantat 1 im dargestellten Ausführungsbeispiel durch das linke Foramen intervertebrale zwischen den beiden Wirbeln 3, 4 eingebracht wurde - bei Sicht auf die Vorderseite des Patienten -, kann es grundsätzlich auch entlang des rechten Foramen intervertebrale 6 eingebracht werden.

Im Folgenden wird das Implantat für den dargestellten Zugang und die dargestellte Anordnung beschrieben; bei Zugang durch das rechte Foramen intervertebrale 6 ist das Implantat spiegelbildlich zu seiner vertikalen der Längsmittelebene L-V (aufgespannt durch die horizontale Längs- und Querachse L, Q; genauer beschrieben im Folgenden) des dargestellten und im Folgenden beschriebenen Implantats ausgebildet.

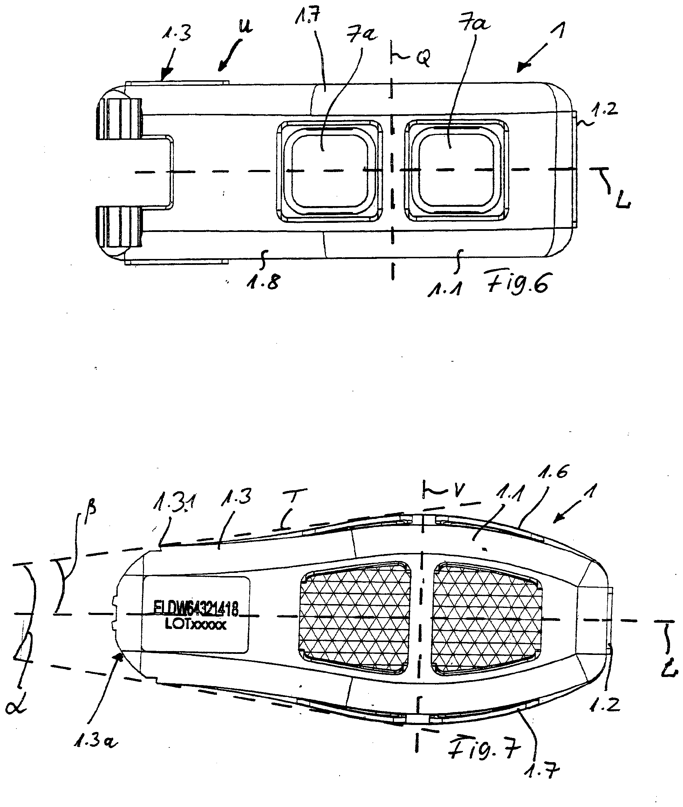

In den Fig. 6-8 sind Längsmittelachse L, horizontale Querachse Q und Vertikalachse V des Implantats 1 darstellt und durch die Darstellungen definiert. Das Implantat 1 hat eine Längserstreckung entlang seiner Längsmittelachse L, die wesentlich größer ist als die Erstreckung in Richtung der Vertikalachse V und der Querachse Q - mindestens eine 1,5-fache Abmessung. Durch die Längsachse L und die Querachse Q wird eine Horizontalmittelebene L-Q bestimmt, durch die Längsachse L und die Vertikalachse V eine Vertikalebene L-V und durch die Querachse Q und die Vertikalachse V eine Querebene Q-V.

Das Implantat weist einen Stützabschnitt 1.1 mit einer distalen Stirnseite 1.2 auf. An dieser schließt sich proximal von einem Übergangsbereich U - entsprechend der Linie U - ein proximaler Angriffsabschnitt 1.3 an, an dem ein Werkzeug zum Einbringen des Implantats 1 in den Zwischenwirbelraum angreifen kann, wie es insbesondere in der DE 20 2013 007 361 U beschrieben ist. Mit einer solchen Winkelausrichtung wird das Implantat 1 in den Wirbelzwischenraum zwischen den Wirbeln 3, 4 in der in dieser Druckschrift beschriebenen Weise durch das - erweiterte - Foramen intervertebrale eingebracht.

Das erfindungsgemäße Implantat 1 ist mit seiner Oberseite 1.6 und Unterseite 1.7 symmetrisch zur Horizontalmittelebene L-Q ausgebildet. Es bzw. sein Stützabschnitt 1.1 weist seine größte Höhe im Bereich der Vertikalmittelachse V bzw. der Vertikalebene V-Q auf, während die Höhe einerseits an der freien distalen Stirnseite 1.1, andererseits im Übergangsbereich U zum Angriffsabschnitt 1.3 gegenüber der Höhe in der vertikalen Mittelebene V-Q geringer ist. In Längsrichtung hat daher das Implantat 1 die Form eines Doppelkeils.

Tangenten T von einer proximalen Kante 1.3.1 des Angriffsabschnitts 1.3 zur proximalen Höhe im Mittelbereich des Tragabschnitts 1.1 weisen dabei zum einen Winkel zueinander zwischen 10° und 20°, vorzugsweise 12° bis 18°, entsprechend zur Längsmittelachse bzw. -Ebene L-Q einen Winkel von 5° bis 10° bzw. 6° bis 9° auf. In Anpassung an den Lordosewinkel zwischen den Wirbeln des Patienten, zwischen die das Implantat 1 eingebracht wird, wird für diesen ein Implantat mit einem ganz spezifischen für ihn geeigneten Winkel α bzw. β gewählt.

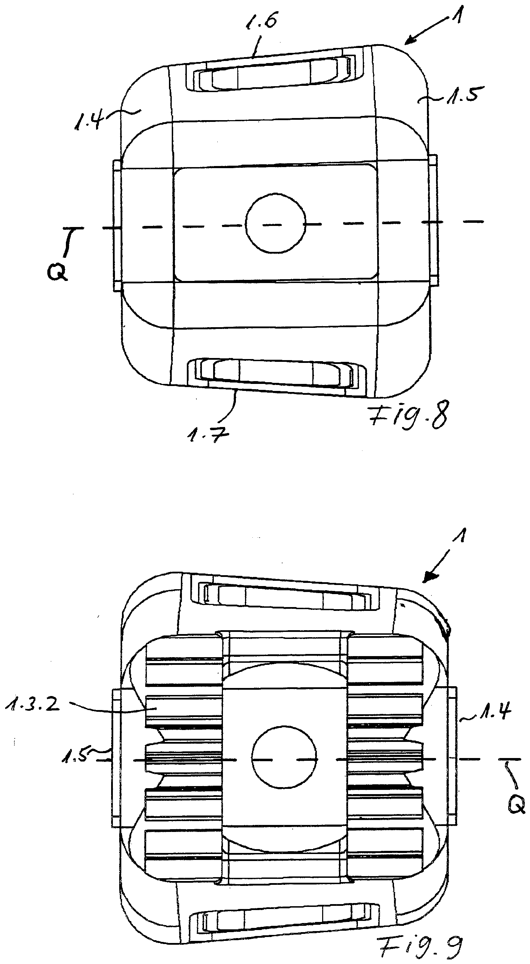

Wie insbesondere den Figuren 8 und 9 entnehmbar ist, ist das Implantat 1 zur Längsvertikalebene L-V asymmetrisch ausgebildet. Es weist im Bereich der - aus Sicht auf die proximale Stirnseite 1.3a - linken vertikalen Längsseite 1.5 eine größere Höhe auf als bei der rechten Längsseite 1.4 (Fig. 9.

Der Höhenunterschied der beiden Längsseiten 1.4, 1.5 in der Mitte des Stützabschnitts 1.1 beträgt dabei jeweils ca. 1,5 mm bis 3 mm, vorzugsweise 2 mm. Durch diesen Höhenunterschied bzw. Neigungen der Oberseite 1.6 und Unterseite 1.7 ist das Implantat besser an die anatomischen Verhältnisse der Unterseite des oberen Wirbels 3 und der Oberseite des unteren Wirbels 4 angepasst, als dies bei paralleler Ausrichtung von Ober- und Unterseite 1.6, 1.7 der Fall wäre. Der guten Ordnung halber sei noch einmal darauf hingewiesen, dass der vorstehend beschriebene Höhenunterschied bzw. die Neigung von Oberseite 1.6 und Unterseite 1.7 sich auf ein durch das in der definierten Weise linke Foramen intervertebrale einzubringendes Implantat bezieht, während ein gegebenenfalls durch das rechte Foramen intervertebrale einzubringendes Implantat demgemäß zur Längsvertikalebene L-V spiegelsymmetrisch ausgebildet ist.

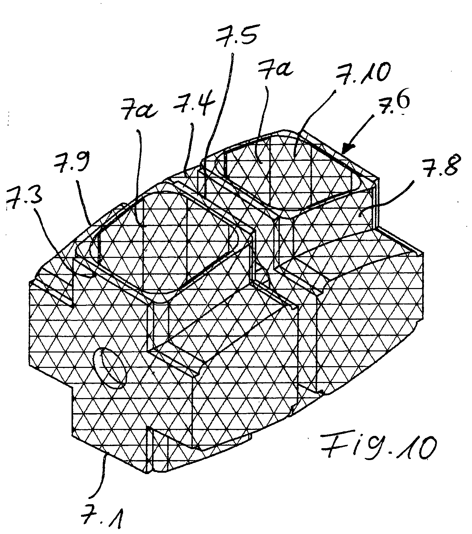

Abgesehen von den beiden beschriebenen Abschnitten Stützabschnitt 1.1 und Angriffsabschnitt 1.3 weist das erfindungsgemäße Implantat die Komponenten eines äußeren starren Rahmens 1.8 und eines gitterförmigen inneren Kerns 1.9 in Form eines Gitterkörpers 7 auf, wie letzterer in der Fig. 10 dargestellt ist. Die Komponenten 1.8 und 1.9 bilden einstückig das Implantat 1. Durch die im äußeren Rahmen 1.8 sichtbaren Durchbrüche in den oberen Wandungen und Längsseitenwandungen kann Knochen in den gitterförmigen inneren Kern 1.9 hineinwachsen, mit diesem verwachsen und damit eine stabile Verbindung ergeben.

Der Gitterkörper 7 weist eine Gitterstruktur, vorzugsweise eine Diamantgitterstruktur mit dünnen Rippen 7.1 und zwischen diesen freien Zwischenräumen auf, wobei die Abmessungen der Rippen 7.1, insbesondere deren Stärke (in senkrechter Richtung zur Erstreckungsrichtung der Rippen 7.1 zwischen zwei Knotenpunkten, an denen sie jeweils mit weiteren Rippen des Gitters verbunden sind) gering ist gegenüber sämtlichen Abmessungen von Strukturkomponenten des Rahmens 1.8, wie beispielsweise einer Breite von Streben des Rahmens 1.8. Die Größenverhältnisse betragen mindestens 1:3. Entsprechendes gilt für die Länge von Streben 7.1 des Gitterkörpers 7 zwischen zwei Knotenpunkten und Längsabmessungen von Strukturteilen des Rahmens 1.8, wie den genannten Rippen, so dass auch hier das Verhältnis mindestens 1:3 beträgt.

Der Rahmen 1.8 weist vier Längsstreben 1.11 auf, die die distale Stirnseite 1.2 des Implantats 1 und den proximalen Angriffsabschnittbereich 1.3 verbinden und mit diesem einen Hohlraum einschließen, in dem sich beim fertigen Implantat der Gitterkörper 7 befindet. Der Gitterkörper weist zwei vertikale Durchbrüche 7a auf. Die Längsstreben 1.11 sind jeweils stirnseitig und mittig, d.h. etwa auf der Hälfte der Länge des Stützabschnitts 1.1 durch Querrippen 1.12 miteinander verbunden. Demgemäß verbleibt zwischen den Querrippen 1.12 ein Durchlass. Entsprechende Durchlässe finden sich auch insbesondere an der distalen Stirnseite 1.2.

In äußerst bevorzugter Ausgestaltung sind Gitterkörper 7 und Rahmen 1.8 nur in senkrecht zur Längsrichtung L und damit an quer verlaufenden (Flächen-)Bereichen miteinander - einstückig - verbunden, wie dies genauer in der DE 20 2013 007 361 U beschrieben ist.

Demgegenüber sind Längsflächen, wie 7.8 und auch Längskanten, wie 7.9 des Gitterkörpers 7 nicht fest mit dem Rahmen 1.8 verbunden. Darüber hinaus entsprechen insbesondere die Abmessungen von oberen Flächen 7.10 und diesen parallel auf der Unterseite der Gitterkörper 7 gegenüberliegenden unteren Flächen den Ausnehmungen bzw. den durch die Längsstreben 1.11, den Querrippen 1.12 und dem Stirnbereich 1.2 freigelassenen Bereichen. Dies führt dazu, dass bei Einwirkung von Druckbelastung auf die Längsrippen des Rahmens 1.8 durch die Wirbelkörper 3, 4 diese nicht an den Längsseiten auf den Gitterkörper 7 übertragen wird und dieser damit unverformt bleibt und seine Aufgabe, ein Einwachsen von Knochenmaterial in diese Waben- oder Zwischenräume des Gitterkörpers 7 zu gewährleisten, auch unter diesen Umständen erfüllen kann. Der durchgehende Längsdurchlass des Implantats 1 ermöglicht, dass dieses über einen liegenden Führungsdraht in das Wirbelzwischenfach eingeführt werden kann.

Der proximale Angriffsabschnitt 1.3 weist an seiner proximalen gerichteten Stirnseite Zähnungen 1.3.2 auf. Diese dienen dazu, beim Festlegen des Implantats 1 an dem distalen Ende eines Einsetzinstruments (DE 20 2013 007 361 U und EP 2 983 622 A2) durch axiales Verspannen zwischen einem hammerartigen Verriegelungsglied des Einsetzinstruments und einem Widerlager desselben eine vorgegebene angulare Ausrichtung zwischen Einsetzinstrument und Implantat 1 zu sichern. Die Zähnungen 1.3.2 sind von auf einem Kreisbogen aufeinanderfolgenden Zähnen gebildet. Es findet sich jeweils eine Zähnung 1.3.2 auf jeder Seite eines proximalen Einlasses 1.3.3 oder der Öffnung des Implantats 1 an der proximalen Stirnseite des Angriffsabschnitts 1.3.

Die Ausgestaltung (z.B. Anzahl, Abstand, Form) der Zähnungen 1.3.1 können auf das Einsetzinstrument abgestimmt sein. Dadurch ist zum einen eine optimale Kompatibilität zum Einsetzinstrument, eine erhöhte Stabilität der Verbindung zwischen Widerlagern des Einsetzinstruments und proximalem Angriffsabschnitt 1.3 des Implantats 1 und zum anderen eine Vielzahl von Verbindungswinkeln ermöglicht.

Das Einbringen eines erfindungsgemäßen Implantats 1 geschieht mittels eines Einsetzinstruments, wie dies in der DE 20 2013 007 361 U und EP 2 983 622 A2 dargestellt und beschrieben ist. Ein Verriegelungselement weist ein um seine Längsachse verdrehbares hammerartiges Verriegelungsteil auf, das in senkrechter Ausrichtung in den Durchlass 1.3.2 des proximalen Angriffsabschnitts 1.3 des Implantats 1 eingeführt wird. Hierzu weist das Implantat 1 an seiner proximalen Stirnseite eine hinterschnittene Öffnung 1.3.3 auf, deren Öffnungsquerschnitt dem Verriegelungselement des Einsetzinstruments entspricht; die hinterschnittene Öffnung bildet ein Verriegelungselement am Implantat 1, wodurch eine Verriegelung von Einsetzinstrument und Implantat 1 ermöglicht wird.

Das hammerartige Verriegelungsteil des Einsetzinstruments wird im Folgenden relativ zu einem vorgesehenen äußeren Rohr um 90° geschwenkt, so dass es in Hinterschneidungen auf der Innenseite der Wandung des proximalen Angriffsabschnitts 1.3 zum Greifen kommt. Mittels Spanneinrichtungen am proximalen Ende des Einsetzinstruments werden das hammerartige Verriegelungsteil und konkav gebogene Stirnkanten an einem distal am äußeren Rohr vorgesehenen Widerlager unter Zwischenlage von zum Verriegelungsteil hintergriffenen am proximalen Angriffsabschnitt 1.3 des Implantats 1 vorgesehenen Zähnungen gegeneinander verspannt. Hierdurch wird das Implantat 1 fest am Einsetzinstrument gehalten. Damit wird ein Bewegen des Implantats 1 in seiner Erstreckungsrichtung und mit einer Komponente zur Erstreckungsrichtung des Einsetzinstruments ermöglicht. Soweit der proximale Angriffsabschnitt 1.3 des Implantats 1 an seiner (äußeren) proximalen Stirnseite eine Zähnung aufweist, ist hierdurch auch eine eingenommene Winkelstellung zwischen Einsetzinstrument und Implantat 1 gesichert. Dennoch ist eine winkelmäßig unterschiedliche Ausrichtung zwischen Einsetzinstrument und Implantat in senkrechter Richtung zur Längserstreckung L von beiden unter beträchtlichen Winkeln von bis zu 30° und mehr möglich.

## Patentansprüche

1. Zwischenwirbel-Implantat (1) mit einem Stützabschnitt (1.1) und mit einem sich an diesen in Längsrichtung anschließenden proximalen Angriffsabschnitt (1.3), wobei eine Oberseite (1.6) und eine Unterseite (1.7) symmetrisch zu einer Horizontalmittelebene (L-Q) ausgebildet sind, **dadurch gekennzeichnet, dass** zwischen Stützabschnitt (1.1) und Angriffsabschnitt (1.3) ein konkaver Übergangsbereich (U) ausgebildet ist, dass die Höhe des Stützabschnitts (1.1) zwischen Übergangsbereich (U) zum Angriffsabschnitt (1.3) und einer dem Angriffsabschnitt (1.3) abgewandten distalen Stirnseite (1.2) größer ist als die Höhe beim Übergangsbereich (U) und an der distalen Stirnseite (1.2) und dass die größte Höhe in der Mitte zwischen dem Übergangsbereich (U) zum Angriffsbereich (1.3) und diesem abgewandter distaler Stirnseite (1.2) ausgebildet ist.

2. Implantat nach Anspruch 1, **dadurch gekennzeichnet, dass** seine Höhe an einer Längsseite (1.5) größer ist als an einer gegenüberliegenden Längsseite (1.4).

3. Implantat nach Anspruch 1 oder 2, **gekennzeichnet durch** einen äußeren Rahmen (1.8) aus massiven Tragteilen und einem inneren Kern (1.9) in Form eines Gitterkörpers (7).

4. Implantat nach einem der Ansprüche 1 bis 3, **dadurch gekennzeichnet, dass** der Gitterkörper (7) nur an parallel in einer Querrichtung verlaufenden Flächen (7.3-7.6) mit dem Rahmen (1.8) verbunden ist, aber in zu diesen Flächen (7.3-7.6) unter einem endlichen Winkel verlaufenden Flächen (7.8) und Kanten (7.9) nicht mit dem Rahmen (1.8) in Verbindung steht und dass insbesondere der die äußere Kontur bestimmende Rahmen (1.8) und der innerhalb desselben befindliche Kern (1.9) einstückig ausgebildet sind.

5. Implantat nach einem der vorangehenden Ansprüche, **dadurch gekennzeichnet, dass** es durch Sintern, wie mittels Lasersintern, hergestellt ist.

6. Implantat nach einem der Ansprüche 1 bis 5, **dadurch gekennzeichnet, dass** es mittels Elektronenstrahlschmelzen, insbesondere selektivem Elektronenstrahlschmelzen, hergestellt ist.

7. Implantat nach einem der Ansprüche 4 bis 6, **dadurch gekennzeichnet, dass** obere und untere Flächen (7.10) des Kerns (1.9) gleiche Abmessungen aufweisen wie von Rahmenkomponenten umgebene Freiräume, die die genannten Flächen (7.10) des Gitterkörpers umfassen.

8. Implantat nach einem der Ansprüche 4 bis 7, **dadurch gekennzeichnet, dass** der Rahmen (1.8) einen Hohlraum umgibt, in dem der als Gitterkörper (7) ausgebildete Kern (1.9) angeordnet ist.

9. Implantat nach einem der Ansprüche 4 bis 8, **dadurch gekennzeichnet, dass** der Rahmen (1.8) in seiner Längsrichtung verlaufende Längsrippen aufweist, wobei insbesondere benachbarte Längsrippen mittig durch Querrippen verbunden sind.

10. Implantat nach einem der Ansprüche 4 bis 9, **dadurch gekennzeichnet, dass** der Gitterkörper (7) eine Diamantstruktur aufweist.

11. Implantat nach einem der Ansprüche 4 bis 10, **dadurch gekennzeichnet, dass** es einen durchgehenden Durchlass entlang einer Längsmittelachse (L) aufweist.

12. Implantat nach einem der Ansprüche 4 bis 11, **dadurch gekennzeichnet, dass** der Gitterkörper (7) Gitter-Öffnungsdurchmesser jeder Öffnung von 0,5 mm bis 3,5 mm aufweisen, wobei insbesondere der Gitterkörper (7) an seiner Außenseite Öffnungsdurchmesser jeder Öffnung in der Größenordnung von 0,5 mm bis 0,7 mm aufweist.

13. Implantat nach einem der vorangehenden Ansprüche, **dadurch gekennzeichnet, dass** der proximale Angriffsbereich (1.3) zur Verbindung des Implantats mit einem Einsetzinstrument auf der Innenseite von Seitenwänden des Implantats hinterschnittene Vertiefungen aufweist, die eine angular bewegliche hinterschneidende Verbindung ermöglicht.

14. Implantat nach einem der vorangehenden Ansprüche, **dadurch gekennzeichnet, dass** der Angriffsbereich (1.3) an der Oberseite einen Ausschnitt aufweist, der eine angulare Beweglichkeit des Implantats (1) ermöglicht.

15. Implantat nach einem der vorangehenden Ansprüche, **dadurch gekennzeichnet, dass** eine proximale Stirnseite des Angriffsbereichs (1.3) Zähnungen aufweist, wobei insbesondere die Zähnungen durch in vertikale Richtung auf Kreisabschnitten aufeinanderfolgend ausgebildet sind sowie die Zähnungen beidseitig eines an der proximalen Stirnseite mittig ausgebildeten proximalen Durchlasses angeordnet sind.

## Claims

1. An intervertebral implant (1) with a support portion (1.1) and with a proximal contact portion (1.3) adjoined thereto in the longitudinal direction, wherein an upper side (1.6) and a lower side (1.7) are formed symmetrically relative to a horizontal center plane (L-Q), **characterized in that** a concave transition area (U) is formed between the support portion (1.1) and the contact portion (1.3), that the height of the support portion (1.1) between the transition area (U) to the contact portion (1.3) and a distal end face (1.2) remote from the contact portion (1.3) is greater than the height at the transition area (U) and at the distal end face (1.2) and that the greatest height is formed in the middle between the transition area (U) to the contact area (1.3) and the distal end face (1.2) remote therefrom.

2. The implant according to claim 1, **characterized in that** the height thereof is greater on one longitudinal side (1.5) than on an opposite longitudinal side (1.4).

3. The implant according to one of the claims 1 or 2, **characterized by** an outer frame (1.8) made of solid supporting parts and an inner core (1.9) in the form of a lattice body (7).

4. The implant according to one of claims 1 to 3, **characterized in that** the lattice body (7) is connected to the frame (1.8) only at surfaces (7.3-7.6) running parallel in a transverse direction, but is not connected to the frame (1.8) in surfaces (7.8) and edges (7.9) which run at a finite angle relative to said surfaces (7.3-7.6) and that in particular the frame (1.8), which determines the outer contour, and the core (1.9) located therewithin are formed in one piece.

5. The implant according to one of the preceding claims, **characterized in that** it is produced by sintering, such as by means of laser sintering.

6. The implant according to one of claims 1 to 5, **characterized in that** it is produced by means of electron beam melting, in particular selective electron beam melting.

7. The implant according to one of claims 4 to 6, **characterized in that** upper and lower surfaces (7.10) of the core (1.9) have the same dimensions as the open spaces surrounded by frame components, said open spaces comprising said surfaces (7.10) of the lattice body.

8. The implant according to one of claims 4 to 7, **characterized in that** the frame (1.8) surrounds a cavity in which the core (1.9) designed as a lattice body (7) is disposed.

9. The implant according to one of claims 4 to 8, **characterized in that** the frame (1.8) has longitudinal ribs running in the longitudinal direction thereof, in particular adjacent longitudinal ribs being connected centrally by transverse ribs.

10. The implant according to one of claims 4 to 9, **characterized in that** the lattice body (7) has a diamond structure.

11. The implant according to one of claims 4 to 10, **characterized in that** it has a continuous passage along a longitudinal central axis (L).

12. The implant according to one of claims 4 to 11, **characterized in that** the lattice body (7) has lattice opening diameters of each opening of 0.5 mm to 3.5 mm, wherein in particular the lattice body (7) has on the outside thereof opening diameters of each opening in the order of magnitude of 0.5 mm to 0.7 mm.

13. The implant according to one of the preceding claims, **characterized in that** the proximal contact area (1.3) for connecting the implant using an insertion instrument has undercut recesses on the inside of side walls of the implant, the recesses enabling an angularly movable undercut connection.

14. The implant according to one of the preceding claims, **characterized in that** the contact area (1.3) has a cutout on the upper side which enables angular mobility of the implant (1).

15. The implant according to one of the preceding claims, **characterized in that** a proximal end face of the contact area (1.3) has serrations, the serrations in particular being formed in succession in the vertical direction on circular sections and the serrations being disposed on both sides of a proximal passage formed centrally on the proximal end face.

## Revendications

1. Implant intervertébral (1) avec une section de maintien (1.1) et avec une section de préhension (1.3) proximale raccordée au niveau de celle-ci dans la direction longitudinale, un côté supérieur (1.6) et un côté inférieur (1.7) étant réalisés symétriquement par rapport à un plan moyen horizontal (L-Q), **caractérisé en ce qu'**une zone de transition (U) concave est réalisée entre la section de maintien (1.1) et la section de préhension (1.3), que la hauteur de la section de maintien (1.1), entre la zone de transition (U) menant à la section de préhension (1.3) et un côté avant (1.2) distal opposé à la section de préhension (1.3), est plus grande que la hauteur dans la zone de transition (U) et au niveau du côté avant (1.2) distal et que la plus grand hauteur est réalisée au centre entre la zone de transition (U) menant à la zone de préhension (1.3) et le côté avant (1.2) distal opposé.

2. Implant selon la revendication 1, **caractérisé en ce que** sa hauteur au niveau d'un côté longitudinal (1.5) est plus grande qu'au niveau d'un côté longitudinal (1.4) opposé.

3. Implant selon la revendication 1 ou 2, **caractérisé par** un cadre extérieur (1.8) composé de parties portantes massives et d'un noyau intérieur (1.9) sous la forme d'un corps grillagé (7).

4. Implant selon l'une quelconque des revendications 1 à 3, **caractérisé en ce que** le corps grillagé (7) est relié au cadre (1.8) uniquement au niveau de surfaces (7.3-7.6) s'étendant parallèlement dans une direction transversale, mais n'est pas relié au cadre (1.8) dans des surfaces (7.8) et arêtes (7.9) s'étendant selon un angle fini par rapport à ces surfaces (7.3-7.6) et que notamment le cadre (1.8) définissant le contour extérieur et le noyau (1.9) se trouvant à l'intérieur de celui-ci sont réalisés d'un seul tenant.

5. Implant selon l'une quelconque des revendications précédentes, **caractérisé en ce qu'**il est fabriqué par frittage, par exemple par frittage par laser.

6. Implant selon l'une quelconque des revendications 1 à 5, **caractérisé en ce qu'**il est fabriqué par fusion par bombardement électronique, notamment par fusion par bombardement électronique sélectif.

7. Implant selon l'une quelconque des revendications 4 à 6, **caractérisé en ce que** les surfaces supérieure et inférieure (7.10) du noyau (1.9) présentent les mêmes dimensions que les espaces libres entourant les composants de cadre et comprenant lesdites surfaces (7.10) du corps grillagé.

8. Implant selon l'une quelconque des revendications 4 à 7, **caractérisé en ce que** le cadre (1.8) ceint un espace creux dans lequel est disposé le noyau (1.9) réalisé sous la forme d'un corps grillagé (7).

9. Implant selon l'une quelconque des revendications 4 à 8, **caractérisé en ce que** le cadre (1.8) comporte des nervures longitudinales s'étendant dans sa direction longitudinale, des nervures longitudinales connexes étant notamment reliées au centre par des nervures transversales.

10. Implant selon l'une quelconque des revendications 4 à 9, **caractérisé en ce que** le corps grillagé (7) présente une structure de diamant.

11. Implant selon l'une quelconque des revendications 4 à 10, **caractérisé en ce qu'**il comporte un passage traversant s'étendant le long d'un axe central longitudinal (L).

12. Implant selon l'une quelconque des revendications 4 à 11, **caractérisé en ce que** le corps grillagé (7) présente un diamètre d'ouverture de grillage pour chaque ouverture allant de 0,5 mm à 3,5 mm, le corps grillagé (7) comportant notamment au niveau de son côté extérieur un diamètre d'ouverture pour chaque ouverture d'un ordre de grandeur de 0,5 mm à 0,7 mm.

13. Implant selon l'une quelconque des revendications précédentes, **caractérisé en ce que** la zone de préhension (1.3) proximale par rapport à la liaison de l'implant comporte des renfoncements découpés par l'arrière avec un instrument de chargement sur le côté intérieur des parois latérales de l'implant qui permet une liaison par détouré arrière mobile sur le plan angulaire.

14. Implant selon l'une quelconque des revendications précédentes, **caractérisé en ce que** la zone de préhension (1.3) comporte un détouré au niveau du côté supérieur qui permet une mobilité angulaire de l'implant (1).

15. Implant selon l'une quelconque des revendications précédentes, **caractérisé en ce qu'**un côté avant proximal de la zone de préhension (1.3) comporte des endentements, les endentements étant notamment réalisés de façon successive de façon traversante dans la direction verticale sur des sections circulaires et les endentements étant disposés des deux côtés d'un passage proximal réalisé de façon centrale au niveau du côté avant proximal.
